# EUROPEAN PATENT SPECIFICATION

(11) **EP 2 119 423 B1**
(45) Date of publication and mention of the grant of the patent: **02.05.2012**
(21) Application number: 08156144.1
(22) Date of filing: 14.05.2008
(51) Int. Cl.: A61J 1/16, A61M 5/00, A61J 7/04

(54) **Storage system for medicine administering device, including reminder means**
Aufbewahrungssystem für eine medizinische Verabreichungsvorrichtung mit Erinnerungsmitteln
Système de stockage pour dispositif d'administration médical, y compris des supports de rappel

(43) Date of publication of application: 18.11.2009
(73) Proprietor: Technical Services Stevens, 3840 Borgloon (BE)
(72) Inventor: Stevens, Danny Technical Services Stevens, 3840 Borgloon (BE)
(74) Representative: Bird, William Edward

(56) References cited:
- EP-A- 1 857 089
- WO-A-99/61324
- WO-A-2004/010231
- DE-A1- 3 824 217

## Description

### Technical field of the invention

The present invention is related to a storage system for a medicine administering device, such as an insulin pen.

### Background of the invention

For a number of medical conditions, the patient is required to administer to him or herself, a dose of medicine at regular intervals. A primary example of such treatment is the administering of insulin injections. It is crucial that a patient maintains a very regular injection pattern, and that the risk of forgetting a dose is minimized. In the case of insulin, the administering device that is mostly used is a so-called insulin pen, wherein a disposable insulin cartridge can be placed. A disposable needle is attached to the pen before injection.

Insulin pens exist which are provided with a memory means for storing the last time of use. A disadvantage of these systems is that the memory means needs to be replaced together with the pen.

Existing reminder devices, like those shown in EP 1857 089 A1 and WO 2004/010231 A2, are linked to or incorporated into a given type of pen, and to one pen only, and cannot be adapted to other pen types or to more than one pen.

### Summary of the invention

The object of the present invention is to provide a universally applicable system suitable for storing any type or number of insulin pens or similar medicine administering devices, including a means for reminding the patient of the last use.

The above objective is accomplished by a system according to the present invention.

The invention is set out in the accompanying independent and dependent claims. Features from the dependent claims may be combined with features of the independent claims and with features of other dependent claims as set out in the claims.

The above and other characteristics, features and advantages of the present invention will become apparent from the following detailed description, taken in conjunction with the accompanying drawings, which illustrate, by way of example, the principles of the invention. This description of illustrative embodiments is given for the sake of example only, without limiting the scope of the invention. The reference figures quoted below refer to the attached drawings.

### Brief description of the drawings

Figure 1 shows a storage system for an insulin pen, according to a first embodiment of the invention.
Figures 2 and 3 show details of the system of figure 1.
Figure 4 shows a storage system according to a second embodiment.
Figure 5 shows a detail of the system of figure 4.
Figure 6 shows the concept of the replaceable inserts in a system of the invention.
Figures 7 and 8 show examples of the digital display in a system according to the invention.
Figure 9 shows another embodiment according to the invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination claimed.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from technical teaching of the invention, the invention being limited only by the terms of the appended claims.

It is to be understood that although preferred embodiments, specific constructions and configurations, as well as materials, have been discussed herein for devices according to the present invention, various changes or modifications in form and detail may be made without departing from the scope of this invention as defined by the appended claims. For example, any formulas given above are merely representative of procedures that may be used. Functionality may be added or deleted from the block diagrams and operations may be interchanged among functional blocks. Steps may be added or deleted to methods described within the scope of the present invention.

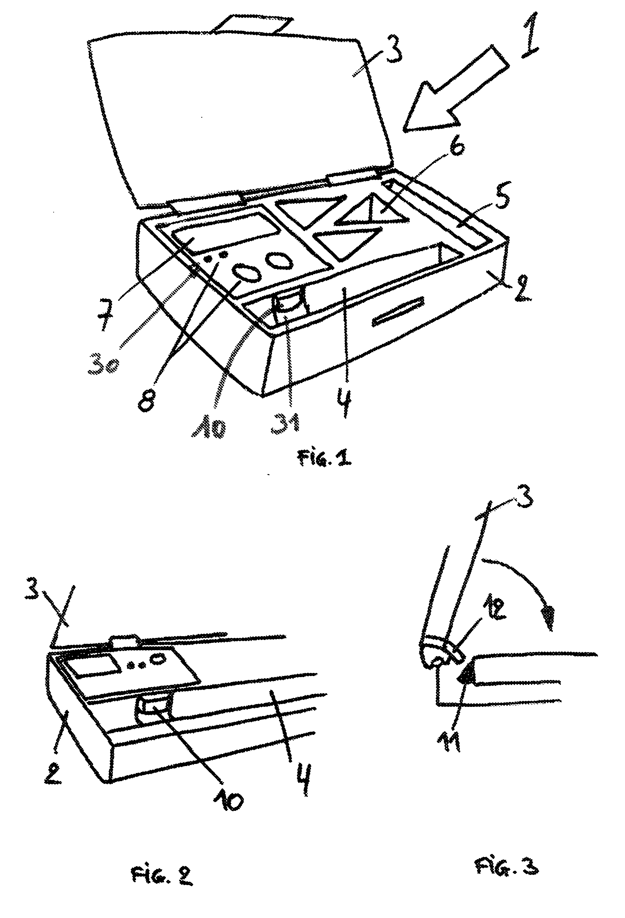

Figure 1 shows a first embodiment of a storage system of the invention. It comprises a container 1, with a box-shaped portion 2 for storing various items, and a hinged lid 3. A first compartment 4 is provided for receiving an insulin pen or the like. A second compartment 5 is provided for receiving a cartridge, and further compartments 6 are provided for storing additional needles. A digital display 7 is incorporated into the container 1, in cooperation with a number of buttons 8 for operating electronic means (circuitry, etc.) equally incorporated in the container.

As seen in figure 2, a switch 10 is arranged in the container. This switch is operable between two states. It can be any type of known electronic switching device (electrical, electronic, optical, etc). The switch is arranged so that it is activated into the first or second of said two states, respectively by inserting or removing a pen from the compartment 4. The switch is connected to the electronic means provided in the container, which comprises memory means for storing a point in time on which the switch is activated in the first or second state. Preferably, the point which is memorized is the moment when the pen is replaced in the container and the lid 3 is closed. The electronic means are further capable of displaying the memorized point in time, for example when the user presses one of the buttons 8. Preferably, the electronic means is arranged to provide an audio signal when the pen is replaced in the receiving compartment, to indicate to the user that the time of replacement has been set by the system.

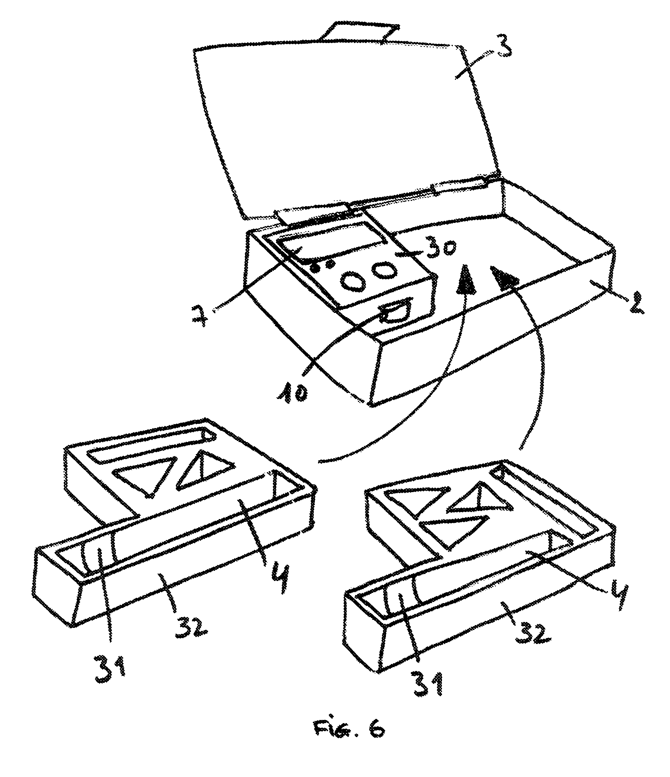

The electronic means may be a separate unit 30 (best visible in figure 6), comprising the display 7 and the necessary operating buttons 8, and also comprising the switch 10. The unit 30 is placed in proximity to the receiving compartment 4, and said compartment 4 may comprise an opening 31, corresponding to the position of the switch 10, said switch protruding through the opening so as to be able to be activated by the insertion of a pen in the receiving compartment 4.

According to the preferred embodiment, the switch 10 is arranged in connection with the pen receiving compartment 4, so as to be able to be activated by one or several types of the pen, said types differing from each other in shape and diameter. As shown in figure 6, the system may be provided with several inserts 32, which each comprise a receiving compartment 4 of a shape and size adapted to a particular type of pen, and/or differing numbers and/or shapes of other compartments (e.g. for needles and cartridges). The provision of these replaceable inserts makes the system universally applicable to any number of pen shapes or types. As shown in figure 6, each insert may be provided with an opening 31, so that the receiving compartment of each insert can be placed in proximity to the electronic unit 30, the switch 10 protruding through the opening 31.

The container is provided with a second switch 11, which is activated by opening the lid. This is illustrated by the detail of figure 3. The lid comprises a finger-like extension 12 at the bottom, which closes the switch 11 when the lid is closed. Likewise, the switch 11 is released when the lid is opened. The switch 11 is equally connected to the electronic means provided in connection with the container. The switch may be comprised in the electronic unit 30, and arranged on the opposite side of said unit 30, opposite the first switch 10. According to the preferred embodiment, when the lid is opened, the digital display lights up automatically, and shows automatically the time at which the pen was last replaced in compartment 4. The above-described actions are performed by the appropriate connectivity and programming of the electronic means in connection with the switches, as is clear to the person skilled in the art of electronics. The second switch 11 is preferably an NO-(Normally Open) contact. The switch 11 may be logically coupled to the switch 10, so that the time of last use is only memorized when both switches are operated, i.e. when they are in a pre-defined state, e.g. when a pen is placed in the holder and the lid is closed.

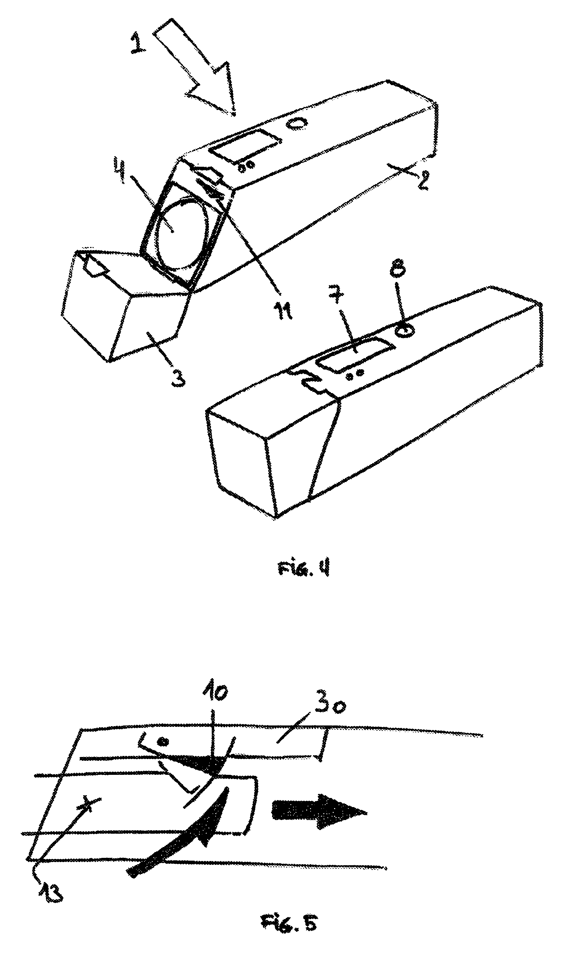

Figure 4 shows a more compact embodiment of the container of the invention. It comprises once again a box-shaped portion 2, a lid 3 and a compartment 4 for receiving a pen. As seen in the detail of figure 5, a switch 10 is provided to be activated into a first or second state by inserting or removing the pen 13 respectively. The display 7 and buttons 8 are equally visible, as is the second switch 11, activated by opening the lid 3. Preferably, the switch 10 (and preferably also switch 11) is part of an electronic unit 30, comprising also the display 7 and buttons 8.

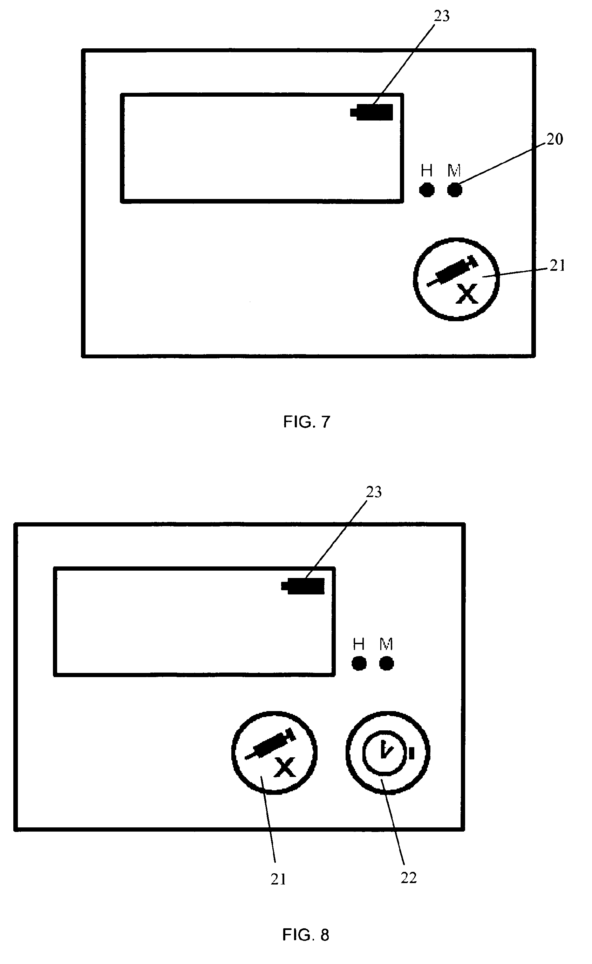

Figure 7 shows a possible embodiment of the unit 30 comprising the display 7 and operating buttons provided in proximity of the display. Two small buttons 20 are provided for setting the current time (expressed in hh:mm - houres:minutes). Preferably, the display will light up when the lid is opened, by the automatic activation of the second switch 11. The time of last use is displayed on the screen, upon which the user may take the pen from the compartment 4 and administer a dose of medicine. Afterwards, the pen is replaced. Either one of the points in time, i.e. when the pen is removed or when it is replaced, or both moments, may be stored in the electronic memory provided in the container. Preferably however, the moment of replacing the pen is recorded.

According to the preferred embodiment, a button 21 is provided, which allows the user to prevent that a moment on which the switch 10 changes its state (e.g. when the pen is replaced) is memorized. This may be done by the following routine:
- the user replaces the pen in compartment 4
- the container shows the moment of replacement of the pen on the screen, for example by flashing the replacement time during an interval of e.g. 20 seconds
- if the user does not want the flashing time to be stored as the time of actual administering of the medicine, he or she presses button 21 and the time is not stored.
- If the user wants to store the flashing time as the actual time of administering, he or she does not press button 21, and the time is stored automatically after the abovementioned interval of e.g. 20s.

This routine allows the user to avoid that every act of replacing the pen is memorized, even when no actual injection has taken place. Sometimes the pen may be taken out and replaced without administering a dose, e.g. for inserting a new cartridge or needle.

According to another embodiment, the display does not light up automatically upon opening the lid, or the display is provided with a sleep mode, so that the display is turned off after a given time. In this way, switch 11 contributes to a power saving in the system of the invention. Also in that case, the button 21 may have the additional function of showing the time of last use on the screen when this button is pressed. An additional button 22 may be provided (fig. 8), to show the actual time on the screen. A battery marker 23 is preferably present, to indicate the lifespan of the battery charge.

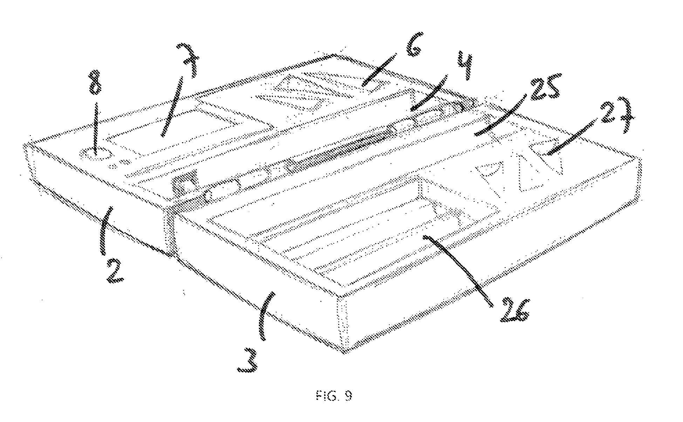

Figure 9 shows another embodiment, wherein the lid 3 is itself provided with a number of compartments. In the embodiment shown, the lid comprises a compartment 25 for an additional pen, and compartments 26 and 27 for additional cartridges and needles respectively. Both compartments 4 and 25 are provided with a switch 10 which can be activated by removal or replacement of a pen. A button may be provided which allows the time of administering of both pens to be displayed on the screen, by repeatedly pressing the button. This embodiment is useful for patients who need to administer different types of medicine during the day. The invention is equally related to other types of systems wherein several pens may be stored (e.g. container of fig. 1 with several receiving compartments for pens in the box-shaped portion 2).

According to one embodiment illustrated by the systems described above, a wire connection (inside unit 30) is present between the container 1 and the display 7, which is preferably incorporated in the container. According to another embodiment, the storage system of the invention comprises a container with a receiving compartment provided with a switch 10, and other components as described above, but wherein the container does not comprise a digital display. The storage system of this embodiment comprises a digital display which is separate from the container, and which is connected to the container by a wireless connection (RF, infra red, bluetooth, etc...). In this embodiment, the digital display may be incorporated in a wristwatch or a cell phone.

In any of the embodiments described above, an additional button may be provided with the following function: by repeatedly pressing the button, several past administering times are displayed on the screen. In this embodiment, the electronic means are adapted to memorize a plurality of moments when the switch 10 is activated.

## Claims

1. A storage system for at least one pen-shaped medicine administering device (13), the system comprising:
- a container (1), the container having a box-shaped portion (2) and a closable lid (3), the container comprising at least one compartment (4), arranged for receiving an administering device,
- a digital display (7),
- a first switch (10), arranged to be activated into a first or second state respectively by inserting or removing said administering device into or from the receiving compartment (4),
- electronic means for memorizing the moment(s) when the administering device is removed and/or replaced, (7),
- a second switch (11) activated by opening the lid (p6: 29-30)
- **characterized by** that when the lid is opened, the last moment of removing the administering device or the last moment of replacing the administering device is displayed by the digital display.

2. The system according to claim 1, wherein said switch (10) is arranged so as to be able to be activated by several types of pen-shaped administering device, said types differing from each other in shape and diameter.

3. The system according to claim 2, provided with one or more replaceable inserts (32) which can be placed in the container, and wherein each insert is adapted to a type of pen-shaped administering device, thereby allowing several types of pen-shaped administering device to be placed in the container and thereby activate the switch (10).

4. The system according to claim 3, wherein each insert comprises a receiving compartment (4) and preferably other compartments, adapted to a particular type and/or shape of pen-shaped administering device.

5. The system according to any one of claims 1 to 4, wherein the display (7) and electronic means are incorporated in a separate unit (30), comprising the switch (10), and wherein said receiving compartment (4) is arranged in proximity to said unit (30).

6. The system according to claim 5, wherein said receiving compartment (4) comprises an opening (31) and wherein the switch (10) protrudes through said opening.

7. The system according to any one of claims 1 to 4, wherein a wireless connection is present between the container (1) and the display (7).

8. The system according to any one of the preceding claims, wherein the further switch (11) is logically coupled to the first switch (10), so that the time of last use is only memorized when both switches are in a pre-defined state.

9. The system according to any one of the preceding claims, comprising a button (21), arranged to allow the user, by pressing this button after replacing the administering device, to prevent the electronic means from memorizing the moment when the administering device was replaced.

10. The system according to any one of the preceding claims, comprising several receiving compartments (4,25) for receiving several pen-shaped administering devices, said receiving compartments being provided in the box-shaped portion (2) and/or in the lid (3).

11. The system according to claim 10, wherein the electronic means are arranged to memorize the times of use of said several devices, and to show said times on the display.

12. The system according to any one of the preceding claims, wherein the electronic means is arranged to memorize several moments at which the administering device has been used, and wherein the system comprises a button arranged to show said several moments on the display, when said button is pressed repeatedly.

## Patentansprüche

1. Lagersystem für mindestens eine stiftförmige Medizinverabreichungsvorrichtung (13), wobei das System umfasst:
- einen Behälter (1), wobei der Behälter einen kastenförmigen Abschnitt (2) und einen schließbaren Deckel (3) aufweist, wobei der Behälter mindestens ein Fach (4) umfasst, das zur Aufnahme einer Verabreichungsvorrichtung ausgebildet ist,
- eine digitale Anzeige (7),
- einen ersten Schalter (10), der zum Aktivieren in einen ersten bzw. zweiten Zustand ausgebildet ist, indem die Verabreichungsvorrichtung in das Aufnahmefach (4) eingesetzt oder aus diesem entfernt wird,
- elektronische Mittel zum Speichern des Moments/der Momente, zu welchen die Verabreichungsvorrichtung entfernt und/oder ausgetauscht wird, (7),
- einen zweiten Schalter, (11), der durch Öffnen des Deckels aktiviert wird (p6: 29-30),
**dadurch gekennzeichnet, dass**, wenn der Deckel geöffnet wird, der letzte Moment des Entfernens der Verabreichungsvorrichtung oder der letzte Moment des Austauschens der Verabreichungsvorrichtung von der digitalen Anzeige angezeigt wird.

2. System nach Anspruch 1, wobei der Schalter (10) so angeordnet ist, dass er durch mehrere Arten von stiftförmiger Verabreichungsvorrichtung aktiviert werden kann, wobei sich die Arten in Form und Durchmesser voneinander unterscheiden.

3. System nach Anspruch 2, das mit einem austauschbaren Einsatz oder mehreren austauschbaren Einsätzen (32) versehen ist, der/die in dem Behälter angeordnet werden kann/können, und wobei jeder Einsatz an eine Art von stiftförmiger Verabreichungsvorrichtung angepasst ist, wodurch mehrere Arten von stiftförmiger Verabreichungsvorrichtung in dem Behälter angeordnet werden können und **dadurch** den Schalter (10) aktivieren.

4. System nach Anspruch 3, wobei jeder Einsatz ein Aufnahmefach (4) und vorzugsweise andere Fächer umfasst, die an eine bestimmte Art und/oder Form von stiftförmiger Verabreichungsvorrichtung angepasst sind.

5. System nach einem der Ansprüche 1 bis 4, wobei die Anzeige (7) und das elektronische Mittel in einer separaten Einheit (30) eingegliedert sind, die den Schalter (10) umfasst, und wobei das Aufnahmefach (4) in der Nähe der Einheit (30) angeordnet ist.

6. System nach Anspruch 5, wobei das Aufnahmefach (4) eine Öffnung (31) umfasst und wobei der Schalter (10) durch die Öffnung ragt.

7. System nach einem der Ansprüche 1 bis 4, wobei eine drahtlose Verbindung zwischen dem Behälter (1) und der Anzeige (7) vorhanden ist.

8. System nach einem der vorangehenden Ansprüche, wobei der weitere Schalter (11) logisch an den ersten Schalter (10) gekoppelt ist, so dass die Zeit der letzten Verwendung nur gespeichert wird, wenn sich beide Schalter in einem vordefinierten Zustand befinden.

9. System nach einem der vorangehenden Ansprüche, umfassend einen Knopf (21), der so angeordnet ist, dass er dem Benutzer ermöglicht, durch Drücken dieses Knopfs nach dem Austauschen der Verabreichungsvorrichtung zu verhindern, dass das elektronische Mittel den Moment speichert, zu dem die Verabreichungsvorrichtung ausgetauscht wurde.

10. System nach einem der vorangehenden Ansprüche, umfassend mehrere Aufnahmefächer (4, 25) zum Aufnehmen mehrerer stiftförmiger Verabreichungsvorrichtungen, wobei die Aufnahmefächer in dem kastenförmigen Abschnitt (2) und/oder in dem Deckel (3) vorgesehen sind.

11. System nach Anspruch 10, wobei die elektronischen Mittel zum Speichern der Verwendungszeitpunkte der mehreren Vorrichtungen und zur Darstellung dieser Zeitpunkte auf der Anzeige angeordnet sind.

12. System nach einem der vorangehenden Ansprüche, wobei das elektronische Mittel zum Speichern mehrerer Momente angeordnet ist, zu welchen die Verabreichungsvorrichtung verwendet wurde, und wobei das System einen Knopf umfasst, der zur Darstellung der mehreren Momente auf der Anzeige angeordnet ist, wenn der Knopf wiederholt gedrückt wird.

## Revendications

1. Système de stockage pour au moins un dispositif d'administration de médicament en forme de stylet (13), le système comprenant :
- un conteneur (1), le conteneur ayant une partie en forme de boîte (2) et un couvercle pouvant être fermé (3), le conteneur comprenant au moins un compartiment (4), agencé pour recevoir un dispositif d'administration,
- un afficheur numérique (7),
- un premier commutateur (10), agencé pour être activé dans un premier ou un second état respectivement en insérant ledit dispositif d'administration dans le compartiment de réception (4) ou en le retirant,
- un moyen électronique pour mémoriser le(s) instant(s) où le dispositif d'administration est enlevé et/ou remplacé, (7),
- un second commutateur (11) activé en ouvrant le couvercle (p6 : 29 - 30)
**caractérisé en ce que**, lorsque le couvercle est ouvert, le dernier instant d'enlèvement du dispositif d'administration ou le dernier instant de remplacement du dispositif d'administration est affiché par l'afficheur numérique.

2. Système selon la revendication 1, dans lequel ledit commutateur (10) est agencé de façon à être susceptible d'être activé par plusieurs types de dispositif d'administration en forme de stylet, lesdits types différant les uns des autres en forme et en diamètre.

3. Système selon la revendication 2, muni d'un ou plusieurs inserts pouvant être remplacés (32) qui peuvent être placés dans le conteneur, et dans lequel chaque insert est adapté à un type de dispositif d'administration en forme de stylet, permettant de ce fait à plusieurs types de dispositif d'administration en forme de stylet d'être placés dans le conteneur et d'activer de ce fait le commutateur (10).

4. Système selon la revendication 3, dans lequel chaque insert comprend un compartiment de réception (4) et de préférence d'autres compartiments, adaptés à un type particulier et/ou à une forme particulière de dispositif d'administration en forme de stylet.

5. Système selon l'une quelconque des revendications 1 à 4, dans lequel l'afficheur (7) et un moyen électronique sont incorporés dans une unité distincte (30), comprenant le commutateur (10), et dans lequel ledit compartiment de réception (4) est agencé à proximité de ladite unité (30).

6. Système selon la revendication 5, dans lequel ledit compartiment de réception (4) comprend une ouverture (31) et dans lequel le commutateur (10) avance à travers ladite ouverture.

7. Système selon l'une quelconque des revendications 1 à 4, dans lequel une connexion sans fil est présente entre le conteneur (1) et l'afficheur (7).

8. Système selon l'une quelconque des revendications précédentes, dans lequel le commutateur supplémentaire (11) est couplé de façon logique au premier commutateur (10), de sorte que l'instant de la dernière utilisation est seulement mémorisé lorsque les deux commutateurs sont dans un état prédéfini.

9. Système selon l'une quelconque des revendications précédentes, comprenant un bouton (21), agencé pour permettre à l'utilisateur, en enfonçant ce bouton après remplacement du dispositif d'administration, d'empêcher le moyen électronique de mémoriser l'instant où le dispositif d'administration a été remplacé.

10. Système selon l'une quelconque des revendications précédentes, comprenant plusieurs compartiments de réception (4, 25) pour recevoir plusieurs dispositifs d'administration en forme de stylet, lesdits compartiments de réception étant prévus dans la partie en forme de boîte (2) et/ou dans le couvercle (3).

11. Système selon la revendication 10, dans lequel le moyen électronique est agencé pour mémoriser les temps d'utilisation desdits plusieurs dispositifs, et pour présenter lesdits temps sur l'afficheur.

12. Système selon l'une quelconque des revendications précédentes, dans lequel le moyen électronique est agencé pour mémoriser plusieurs instants auxquels le dispositif d'administration a été utilisé, et dans lequel le système comprend un bouton agencé pour présenter lesdits plusieurs instants sur l'afficheur, lorsque ledit bouton est enfoncé à plusieurs reprises.
